# EUROPEAN PATENT SPECIFICATION

(11) **EP 4 493 286 B1**
(45) Date of publication and mention of the grant of the patent: **06.05.2026**
(21) Application number: 23711086.1
(22) Date of filing: 16.03.2023
(51) Int. Cl.: A61Q 19/10, A61K 8/368, A61K 8/41, A61K 8/365

(54) **A SOLID CLEANSING COMPOSITION**
FESTE REINIGUNGSZUSAMMENSETZUNG
COMPOSITION DE NETTOYAGE SOLIDE

(30) Priority: 16.03.2022 EP 22162397
(43) Date of publication of application: 22.01.2025
(73) Proprietor: Unilever IP Holdings B.V., 6708 WH Wageningen (NL); Unilever Global IP Limited, Wirral, Merseyside CH62 4ZD (GB)
(72) Inventor: BARNE, Sameer Keshav, 6708 WH Wageningen (NL); DASGUPTA, Anindya, 6708 WH Wageningen (NL); SAJI, Maya Treesa, 6708 WH Wageningen (NL)
(74) Representative: Unilever Patent Group
(86) International application number: PCT/EP2023/056697
(87) International publication number: WO 2023/175057

(56) References cited:
- WO-A1-2018/121946
- RU-C1- 2 456 022
- US-A1- 2003 089 891

## Description

### Field of the invention

The present invention relates to a cleansing composition, related methods and uses, according to the appended claims.

### Background of the invention

WO2018 121946 (Unilever) discloses an antimicrobial composition at the pH of skin. The composition comprises (a) 5 to 40% by weight of an ammonium salt having at least one covalently bonded hydrogen attached to the nitrogen or of an anionic surfactant with ammonium group as counter ion or mixtures thereof; and (b) 0.1 to 20% by weight of a carboxylic acid with pKa value greater than 4.5. See also RU2456022 C1, US 2003/089891 A1, WO 2018/121946 A1.

However, the carboxylic acids discussed in the prior art are those with pKa greater than 4.5. Although they provide good antimicrobial efficacy in presence of anionic surfactants system as discussed therein, these acids are medium chain fatty acids that are costly and are associated with odor which is less preferred. Such odor then needs to be tackled with fragrance system which further increases the costs.

Therefore, there is need to provide a cleansing composition that overcomes one or more drawbacks of prior art.

It has now been found that when carboxylic acids having pKa in the range from 2.5 to 4.5 e.g. benzoic acid, which are easier to incorporate in cleansing compositions without giving rise to odor issues, were combined with ammonium salts e.g. ammonium chloride, synergistic antimicrobial effect was obtained. Moreover, the antimicrobial effect is obtained in short duration like 10 to 30 seconds, e.g. 20 seconds.

### Summary of the invention

In a first aspect, the present invention relates to a solid cleansing composition comprising:
a. from 2 to 15 wt% ammonium salt selected from ammonium chloride, ammonium benzoate, ammonium citrate, ammonium carbonate, ammonium acetate, ammonium sulphate, isopropyl ammonium chloride and mixtures thereof; and
b. from 2 to 25 wt% carboxylic acid having pKa in the range from 2.5 to 4.5,
wherein the pH of 5% solution of the composition is in the range from 2.5 to 6.5.

In a second aspect, the present invention relates to a method of providing antimicrobial effect comprising the steps:
a. applying the composition of the first aspect on to a surface; and
b. optionally, rinsing the surface on to which the composition is applied.

In a third aspect, the present invention relates to use of the composition of the first aspect in providing antimicrobial effect.

In a fourth aspect, the present invention relates to use of an ammonium salt as an enhancer of the antimicrobial efficacy of a solid cleansing composition, said cleansing composition comprising carboxylic acid having pKa in the range from 2.5 to 4.5; and wherein the pH of 5% solution of the composition is in the range from 2.5 to 6.5.

### Detailed description of the invention

Any feature of one aspect of the present invention may be utilized in any other aspect of the invention. The word "comprising" is intended to mean "including" but not necessarily "consisting of" or "composed of." In other words, the listed steps or options need not be exhaustive. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format " x to y" are understood to include x and y. When for a specific feature multiple preferred ranges are described in the format "x to y", it is understood that all ranges combining the different endpoints are also contemplated. Unless specified otherwise, amounts as used herein are expressed in percentage by weight based on total weight of the composition and is abbreviated as "wt%". The use of any and all examples or exemplary language e.g. "such as" provided herein is intended merely to better illuminate the invention and does not in any way limit the scope of the invention otherwise claimed.

For the purpose of the present invention, the terms 'antimicrobial benefit' or 'antimicrobial effect' or 'antimicrobial efficacy', may be used interchangeably and they denote the same meaning. Preferably, 'antimicrobial benefit' or 'antimicrobial effect' or 'antimicrobial efficacy' means reduction in number of microbes e.g. bacteria, fungi, viruses. Such antimicrobial effect is obtained when the composition of the present invention is preferably applied on to a surface that may be an inanimate or animate surface. More preferably, the composition is applied to an animate substrate e.g. human skin. 'Skin' as used herein, preferably means to include skin on any part of the body e.g. face, neck, chest, back, arms, underarms, hands, legs, buttocks and scalp.

In a first aspect, the present invention relates to a solid cleansing composition comprising:
a. from 2 to 15 wt% ammonium salt selected from ammonium chloride, ammonium benzoate, ammonium citrate, ammonium carbonate, ammonium acetate, ammonium sulphate, isopropyl ammonium chloride and mixtures thereof; and
b. from 2 to 25 wt% carboxylic acid having pKa in the range from 2.5 to 4.5, wherein the pH of 5% solution of the composition is in the range from 2.5 to 6.5.

The composition according to the present invention (the composition) is a solid cleansing composition. Preferably, the composition is in the form of a bar. Alternatively, the composition may be in powder form or may be in the form of a concentrated powder.

### Ammonium salts

Generally, salts are added to cleansing compositions as electrolytes to provide the right ionic strength.

The composition comprises ammonium salts selected from ammonium chloride, ammonium benzoate, ammonium citrate, ammonium carbonate, ammonium acetate, ammonium sulphate, isopropyl ammonium chloride or mixtures thereof.

Preferably, the ammonium salts are selected from ammonium chloride, ammonium citrate and mixtures thereof.

The composition comprises from 2 to 15 wt%, preferably from 3 to 13 wt%, more preferably from 4 to 12 wt%, even more preferably from 5 to 11 wt%, further more preferably from 6 to 10 wt%, even further more preferably from 7 to 9 wt%, still more preferably from 8 to 9 wt%, of the ammonium salts described above.

### Carboxylic acids

The composition comprises one or more carboxylic acids having pKa in the range from 2.5 to 4.5. Preferably, the composition comprises one or more carboxylic acids having pKa in the range from 2.5 to less than 4.5. More preferably, the composition comprises one or more carboxylic acids having pKa in the range from 2.5 to 4.4.

Preferably, the one or more carboxylic acids is selected from aromatic carboxylic acids, cyclic carboxylic acids, aliphatic carboxylic acids and mixtures thereof.

Examples of aromatic carboxylic acids include benzoic acid (pKa = 4.2), 2-hydroxy benzoic acid (also known as salicylic acid; pKa = 2.97, 3-hydroxybenzoic acid (also known as m-salicylic acid; pKa = 3.82), 4-hydroxybenzoic acid (pKa = 4.5).

Examples of aliphatic carboxylic acids that may be present in the composition include citric acid (pKa = 3.13), lactic acid (pKa = 3.86), L (+) tartaric acid (pKa = 2.89), meso tartaric acid (pKa = 3.22), glycolic acid (pKa = 3.6) and mixtures thereof.

More preferably, the one or more carboxylic acids is selected from aromatic carboxylic acid, aliphatic carboxylic acid and mixtures thereof. Even more preferably, the one or more carboxylic acids is selected from aromatic carboxylic acid. Further more preferably, benzoic acid is selected as the carboxylic acid.

The composition comprises from 2 to 25 wt%, preferably from 3 to 22 wt%, more preferably from 5 to 20 wt%, even more preferably from 7 to 17 wt%, further more preferably from 9 to 15 wt%, still more preferably from 10 to 14 wt%, yet more preferably from 12 to 13 wt% of one or more carboxylic acid having pKa in the range from 2.5 to 4.5 described above.

It has been found that the ammonium salt as described above in combination with carboxylic acids described above, provides synergistic antimicrobial effect.

### Anionic surfactants:

Preferably, the composition further comprises anionic surfactants. Preferably, the anionic surfactants are selected from alkyl sulfates, alkyl sulfosuccinate, alky sulfoacetate, alkyl ether sulfate, isethionate, taurate; and mixture thereof.

Examples of alkyl sulfates that may be used as anionic surfactant in the composition include sodium lauryl sulfate (SLS), sodium myristyl sulfate and mixtures thereof.

Examples of alkyl sulfosuccinate that may be used as an anionic surfactant includes disodium lauryl sulfosuccinate.

Examples of alkyl sulfoacetate include sodium sulfoacetate. Examples of isethionate include sodium isethionate. Examples of taurates include sodium taurate.

Preferably, the composition comprises from 10 to 80 wt%, more preferably from 15 to 70 wt%, even more preferably from 20 to 60 wt%, still more preferably from 25 to 50 wt% and further more preferably from 30 to 40 wt% anionic surfactants.

### Amphoteric surfactants

Amphoteric surfactants provide improved foam when added in the composition.

Preferably, the composition further comprises amphoteric surfactants. Preferably, amphoteric surfactants are selected from cocamidopropyl betaine (CAPB), cocamide monoethanolamide (CMEA), cocoamphoacetate and mixtures thereof.

Preferably, the composition comprises from 0.1 to 10 wt%, more preferably from 0.5 to 8 wt%, even more preferably from 1 to 7 wt%, further more preferably from 2 to 5 wt% and still more preferably from 3 to 5 wt% amphoteric surfactants.

### Essential oils

Preferably the composition further comprises one or more essential oils. These essential oils may be selected from thymol, terpineol, carvacrol, eugenol, menthol and mixtures thereof. More preferably, the essential oils may be selected from thymol, terpineol, carvacrol, eugenol and mixtures thereof. Even more preferably, the essential oils may be selected from thymol, terpineol, eugenol and mixtures thereof. Further more preferably, the essential oils may be selected from thymol, terpineol and mixtures thereof. In such cases, it is preferred that the w/w ratio of the amount of thymol to that of terpineol is 1:0.1 to 1:10. More preferably the ratio is 1 :0.5 to 1:2.

Preferably, the composition comprises from 0.0001 to 5 wt%, more preferably 0.001 to 2 wt%, even more preferably from 0.01 to 1 wt% and further more preferably from 0.05 to 0.1 wt% one of more of these essential oils.

### pH of the composition

The pH of the 5% solution of the composition is in the range from 2.5 to 6.5, preferably, from 3.0 to 6, more preferably, from 3.5 to 5.5 and further more preferably from 4.0 to 4.5. Preferably, the 5% solution of the composition is prepared using demineralized water.

### Structurants

Preferably, the composition comprises a structurant. Preferably, structurants are, a structurant is selected from silica, talc, calcite, pumice, tricalcium phosphate, chalk, fullers earth, kaolin, starch, gums, silica, sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate and fatty acids having carbon chain from C10 to C20 e.g. lauric acid, myristic acid, palmitic acid, stearic acid and oleic acid; and mixtures thereof.

Talc is a magnesium silicate mineral material, with a sheet silicate structure and a composition of Mg₃Si₄(OH)₂₂ and may be available in the hydrated form. It has a plate-like morphology, and is essentially oleophilic/hydrophobic, i.e., it is wetted by oil rather than water.

Calcium carbonate or chalk exists in three crystal forms: calcite, aragonite and vaterite. The natural morphology of calcite is rhombohedral or cuboidal, acicular or dendritic for aragonite and spheroidal for vaterite. Commercially, calcium carbonate or chalk known as precipitated calcium carbonate is produced by a carbonation method in which carbon dioxide gas is bubbled through an aqueous suspension of calcium hydroxide. In this process the crystal type of calcium carbonate is calcite or a mixture of calcite and aragonite.

Starch may be used directly or modified during the process of making the bar composition such that the starch becomes gelatinized, either partially or fully gelatinized.

Another suitable starch is pre-gelatinized which is starch that has been gelatinized before it is added as an ingredient in the present bar compositions. Various forms are available that will gel at different temperatures, e.g., cold water dispersible starch. An example of pre-gelatinized starch available commercially, is FARMAL^{®} CS 3400.

Preferably the composition comprises structurants in an amount ranging from 1 to 50 wt%, more preferably from 3 to 45 wt%, even more preferably from 5 to 30 wt% and further more preferably from 10 to 25 wt%.

### Polymers

Preferably, the composition further comprises water soluble polymers. Preferably, such polymers are selected from polyvinyl alcohol, polyvinyl pyrrolidone, sodium carboxymethylcellulose, polyethylene glycol, methyl cellulose, hydroxypropyl methyl cellulose and mixtures thereof.

Preferably, the composition comprises from 1 to 20 wt%, more preferably from 3 to 17 wt%, even more preferably from 5 to 15 wt%, further more preferably from 7 to 13 wt% and still more preferably from 9 to 12 wt% water soluble polymer.

Such polymer may be added in the composition mix or may be used to coat the composition. For example, if the composition is in the form of a bar or a tablet, such polymers may form an outer water-soluble coating on such bars or tablets.

### Format of the composition:

The composition is in solid form. Preferably, the composition is formulated in the form of a bar. In such case, bars may be manufactured using known methods like milling and plodding route. Alternatively, the composition may be formulated in the form of a powder or a concentrated powder. In such case, spray drying technology may be employed for manufacture of such powders.

If the composition is formulated in the form of a bar, then it preferably comprises a polyhydric alcohol (also called polyol) or mixture of polyols. Polyol is a term used herein to designate a compound having multiple hydroxyl groups (at least two, preferably at least three) which is highly water soluble, preferably freely soluble, in water. Many types of polyols are available including relatively low molecular weight short chain polyhydroxy compounds such as glycerol and propylene glycol; sugars such as sorbitol, manitol, sucrose and glucose; modified carbohydrates such as hydrolyzed starch, dextrin and maltodextrin, and polymeric synthetic polyols such as polyalkylene glycols, for example polyoxyethylene glycol (PEG) and polyoxypropylene glycol (PPG). Especially preferred polyols are glycerol, sorbitol and their mixtures. Most preferred polyol is glycerol. A bar according to the present invention preferably comprises from 0 to 8 wt%, more preferably from 1 to 7.5 wt% polyol.

Preferably the composition further comprises an opacifier. When opacifiers are present, the bar is generally opaque. Examples of opacifiers include titanium dioxide, zinc oxide and the like. An alternative opacifying agent is zinc stearate. The bar may take the form of a water-clear, i.e. transparent bar, in which case it does not contain an opacifier.

A preferred bar may additionally include up to 30 wt% benefit agents. Preferred benefit agents include moisturizers, emollients, sunscreens and anti-ageing compounds. These agents may be added at an appropriate step during the process of making the bars.

If the composition is formulated in the form of a bar then it preferably comprises water in an mount from 10 to 20 wt%, more preferably from 14 to 18 wt%.

Alternatively, the composition may be formulated in a powder form or a concentrated powder form. In such case, the composition is in dry form where it preferably comprises from 1 to 5 wt%, more preferably from 1 to 3 wt% and even more preferably from 1 to 2 wt% water. Such dry concentrated powder is a rehydratable powder which may be a free-flowing powder or may be in the form of a tablet; and may be packed e.g. into suitable pouches or sachets. If formulated in the form of a tablet, it may optionally be coated with water soluble polymers e.g. polyvinyl alcohol, polyvinyl pyrollidone, sodium carboxycellulose, polyethylene glycol, methyl cellulose, hydroxypropyl methyl cellulose. Such rehydratable powder is preferably rehydrated by an end-user where an appropriate amount of water e.g. 100 mL, to an appropriate amount of the concentrated powder e.g. 5 grams, to prepare a liquid cleansing composition e.g. handwash and bodywash. An appropriate container preferably equipped with a pump may be supplied along with such powder or concentrated powder that can be used to prepare such liquid compositions.

The composition may further comprise one or more of the following ingredients:
The composition may preferably further comprise a skin-lightening agent selected from aloe extract, ammonium lactate, arbutin, azelaic acid, kojic acid, butyl hydroxy anisole, butyl hydroxy toluene, citrate esters, 3 diphenyl propane derivatives, ellagic acid, fennel extract, gluco pyranosyl-1-ascorbate, gluconic acid, glycolic acid, hydroquinone, 4 hydroxyanisole, linoleic acid, magnesium ascorbyl phosphate, 2,4 resorcinol derivatives, 3,5 resorcinol derivatives, vitamins like vitamin B6, vitamin B12, vitamin C, vitamin A, resorcinol derivatives and mixtures thereof.

The composition may preferably further comprise preservatives to protect the composition against the growth of potentially harmful microorganisms. Particularly preferred preservatives include hydantoin derivatives, propionate salts, a variety of quaternary ammonium compounds, phenoxyethanol, imidazolidinyl urea, sodium dehydroacetate and benzyl alcohol. Preservatives are preferably used in amounts ranging from 0.01 wt% to 2 wt%. Alternatively, preservatives may be used in an amount from 2 to 8 wt%, more preferably from 3 to 6 wt% in case the composition is in the form of a concentrated powder.

Diluents other than water can include liquid or solid emollients, solvents, humectants, thickeners and powders. Examples of each of these types of vehicle, which can be used singly or as mixtures of one or more vehicles, are as follows:
Emollients, such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate.

Further, the composition preferably comprises other optional ingredients like anti-oxidants, perfumes, polymers, chelating agents, colourants, deodorants, dyes, emollients, moisturizers, enzymes, foam boosters, lathering agents, skin conditioners, pH adjusters and stabilizers may be added in suitable amounts. Preferably, these ingredients are added after the saponification step. Sodium metabisulphite, ethylene diamine tetra acetic acid (EDTA), borax or ethylene hydroxy diphosphonic acid (EHDP) are preferably added to the formulation. Such ingredients may be present in an amount from 0 to 7 wt%, more preferably from 1 to 5 wt% and even more preferably from 2 to 4 wt% in the composition.

In a second aspect, the present invention also discloses a method of providing antimicrobial effect comprising the steps:
(a) applying a composition according to the invention on to a surface; and
(b) optionally, rinsing the surface on to which the composition is applied.

Step (a) involves applying the composition of the first aspect on to a surface. The surface is preferably an inanimate or animate surface. More preferably, the surface is an animate surface e.g. human skin. 'Skin' as used herein, preferably means to include skin on any part of the body e.g. face, neck, chest, back, arms, underarms, hands, legs, buttocks and scalp.

Step (a) is preferably carried out where a user applies the composition of the first aspect, on to a surface along with water in an amount sufficient to create lather.

The method optionally but preferably, comprises an additional step, step (b) where the surface on to which the composition was applied, is rinsed with water to, at least partially, remove the composition from the surface. Preferably, step (b) of at least partially removing the composition is carried out within less than 5 minutes, more preferably in less than 3 minutes, even more preferably in less than 1 minute and furthermore preferably in less than 35 seconds e.g. 10, 20 or 30 seconds, after step (a). Preferably, the method is non-therapeutic and/or cosmetic in nature.

If the composition is in the form of a concentrated powder, then preferably, an appropriate amount of the concentrated powder e.g. 5 grams is mixed with an appropriate amount of a diluent, preferably water e.g. 100 mL before carrying out step (a). It will also be understood that such step of rehydrating the concentrated powder or performing dilution will be required to be carried once; and thereafter only when such 100 mL liquid cleansing composition will be used by an end-user. Such dilution may be carried out in any container e.g. a bottle, preferably equipped with a stopper or a cap to close the container; and volume of which is enough to hold the amount after dilution e.g. 100 mL. Preferably, a container that is suitable for this rehydration step may be supplied along with the concentrated powder. More preferably, such container is equipped with a dispensing mechanism that is equipped with a pump for easy dispensing of the liquid cleansing composition so formed. A container may first be filled with a diluent such as water and then the concentrated powder composition is added or vice versa. It is preferred that after the addition of the concentrated powder and the diluent liquid a suitable container, the container is closed and shaken for about 15 to 60 seconds. It is preferred that the newly reconstituted liquid composition is allowed to rest for at least 30 minutes and preferably for at least 1 to 2 hrs.

In the method of the second aspect, such liquid composition so prepared is applied on to a surface as described in step (a) above, which is optionally but preferably followed by step (b) as described above.

In a third aspect, the present invention relates to use of a composition described in the first aspect for providing antimicrobial e.g antibacterial, benefit. The composition of the present invention provides antimicrobial effect. Preferably, the use is non-therapeutic and/or cosmetic in nature.

In a fourth aspect, the present invention relates to use of an ammonium salt as an enhancer of the antimicrobial efficacy of a solid cleansing composition, said cleansing composition comprising carboxylic acid having pKa in the range from 2.5 to 4.5; and wherein the pH of 5% solution of the composition is in the range from 2.5 to 6.5. Preferably, the use is non-therapeutic and/or cosmetic in nature.

The various features of the present invention referred to in individual sections above apply, as appropriate, to other sections mutatis mutandis. Consequently, features specified in one section may be combined with features specified in other sections as appropriate. Any section headings are added for convenience only and are not intended to limit the disclosure in any way.

The invention is now further illustrated through the following non-limiting examples.

### Examples

### Protocols

The following protocol was used to evaluate biocidal activity.
IN-VITRO TIME-KILL PROTOCOL - ASTM 2783

### Preparation of the bacterial culture:

For these experiments, *Escherichia.coli* ATCC 10536 was used in the study, which represents gram-negative bacteria. The bacteria were grown overnight on Tryptic soya agar (TSA) plate. The bacterial cell density was then adjusted at 620 nm to a pre-calibrated optical density to get the final count of 10⁹ cfu/mL in saline (0.86% NaCl) by using a spectrophotometer.

### Assay Protocol:

9.9 mL of the composition of different examples (as stated in table 1 below) was taken in different sample containers at specific product dilution. To each of those container 0.1 mL of bacterial culture was added just before performing the assay and mixed well to obtain a mixture. A timer was started immediately after the addition of the culture. The mixture was kept for a specific contact for a duration in from 10 seconds to 30 seconds e.g. 20 seconds.

At the end of each contact time, the antibacterial activity of the samples was neutralized immediately, by addition of 1 mL each of the above mixture to 9 mL of an appropriate neutralizing broth which is validated for the test system. The neutralized samples were then serially diluted up to 5 dilution in neutralizer broth and plated on TSA (40gpL - Difco) in duplicates.

The log reduction was calculated by comparing with the bacterial control. The bacterial control used for this purpose was a mixture prepared by addition of 0.1 mL of bacterial culture to 9.9 mL of saline; the mixture was then serially diluted and plated on TSA. After solidification of the TSA plates, the plates were incubated at 37°C for 48 hours. The colonies on the plates were counted.

Log reduction greater than 5 means 99.99% reduction in the number of CFU and log reduction less than 0.5 means no reduction in the number of CFU that means no antimicrobial efficacy. Log reduction greater than 5 also denotes complete kill.

Powdered samples of carboxylic acids, surfactants and ammonium salts in amounts shown in tables below, were mixed in mortar and pestle. Thereafter, structurants and polymer, if any, in amounts shown in tables below, were added.

The compositions as shown in table 1 below were prepared.

**Table 1**

| **Ingredients (wt%)** | **1** | **A** | **B** |
|---|---|---|---|
| Sodium lauryl Sulfate | 64 | 64 | 64 |
| Benzoic acid | 20 | 20 | - |
| Ammonium Chloride | 10 | - | - |
| Sodium Chloride | - | 10 | 20 |
| Xanthan Gum | 4 | 4 | 4 |
| Silica | 2 | 2 | 12 |
| Total | 100 | 100 | 100 |
| pH of 5% solution | 4.08 | 4.05 | 3.96 |
| log reduction of E. coli ATCC 10536 at 20 sec | >5 | 3.18 | 0.89 |

The data above shows that the composition as per the present invention (example 1) shows synergistic antimicrobial effect.

## Claims

1. A solid cleansing composition comprising:
a. from 2 to 15 wt% ammonium salt selected from ammonium chloride, ammonium benzoate, ammonium citrate, ammonium carbonate, ammonium acetate, ammonium sulphate, isopropyl ammonium chloride and mixtures thereof; and
b. from 2 to 25 wt% one or more carboxylic acids having pKa in the range from 2.5 to 4.5;
wherein the pH of 5% solution of the composition is in the range from 2.5 to 6.5.

2. The composition according to claim 1 wherein the ammonium salt is selected from ammonium chloride, ammonium citrate and mixtures thereof.

3. The composition according to any one of claims 1 or 2 wherein the carboxylic acid is selected from selected from aromatic carboxylic acids, cyclic carboxylic acids, aliphatic carboxylic acids and mixtures thereof.

4. The composition according to any one of claims 1 to 3 wherein the carboxylic acid is selected from an aromatic carboxylic acid selected from benzoic acid, 2-hydroxybenzoic acid, 3-hydroxybenzoic acid, or an aliphatic carboxylic acid selected from citric acid, lactic acid; and mixtures thereof.

5. The composition according to any one of claims 1 to 4 wherein the carboxylic acid is benzoic acid.

6. The composition according to any one of claims 1 to 5 wherein the composition further comprises from 10 to 80 wt% anionic surfactant.

7. The composition according to claim 6 wherein the anionic surfactant is selected from alkyl sulfate, alkyl sulfosuccinate alky sulfoacetate, alkyl ether sulfate, isethionate, taurateand mixtures thereof.

8. The composition according to any one of claims 1 to 7 wherein the composition further comprises one or more essential oils selected from thymol, terpineol, carvacrol, eugenol, menthol and mixtures thereof.

9. The composition according to claim 8 wherein the composition further comprises a structurant selected from silica, talc, kaolinite, bentonite, starch, cellulose, feldspar, mica and mixtures thereof.

10. The composition according to any one of claims 1 to 9 wherein the composition is the form of a bar.

11. The composition according to any one of claims 1 to 9 wherein the composition is in the form of a powder or a concentrated powder.

12. Non therapeutic method of providing antimicrobial effect comprising the steps:
a. applying the composition as claimed in any one of claims 1 to 11 on to a surface; and
b. optionally, rinsing the surface on to which the composition is applied.

13. The method as claimed in claim 12 wherein the step of rinsing the surface is carried out within 5 minutes from applying the composition to the surface.

14. Non therapeutic use of the composition as claimed in any one of the claims 1 to 11 for providing antimicrobial effect.

15. Use of an ammonium salt as an enhancer of the antimicrobial efficacy of a solid cleansing composition, said cleansing composition comprising carboxylic acid having pKa in the range from 2.5 to 4.5; and wherein the pH of 5% solution of the composition is in the range from 2.5 to 6.5.

## Patentansprüche

1. Feste Reinigungszusammensetzung, umfassend:
a. 2 bis 15 Gew.-% Ammoniumsalz, ausgewählt unter Ammoniumchlorid, Ammoniumbenzoat, Ammoniumcitrat, Ammoniumcarbonat, Ammoniumacetat, Ammoniumsulfat, Isopropylammoniumchlorid und Mischungen davon; und
b. 2 bis 25 Gew.-% einer oder mehrerer Carbonsäuren mit einem pKa-Wert im Bereich von 2,5 bis 4,5;
wobei der pH-Wert einer 5%-igen Lösung der Zusammensetzung im Bereich von 2,5 bis 6,5 liegt.

2. Zusammensetzung nach Anspruch 1, wobei das Ammoniumsalz unter Ammoniumchlorid, Ammoniumcitrat und Mischungen davon ausgewählt ist.

3. Zusammensetzung nach irgendeinem der Ansprüche 1 oder 2, wobei die Carbonsäure unter aromatischen Carbonsäuren, cyclischen Carbonsäuren, aliphatischen Carbonsäuren und Mischungen davon ausgewählt ist.

4. Zusammensetzung nach irgendeinem der Ansprüche 1 bis 3, wobei die Carbonsäure ausgewählt ist unter einer aromatischen Carbonsäure, ausgewählt unter Benzoesäure, 2-Hydroxybenzoesäure, 3-Hydroxybenzoesäure oder einer aliphatischen Carbonsäure, ausgewählt unter Zitronensäure, Milchsäure, und Mischungen davon.

5. Zusammensetzung nach irgendeinem der Ansprüche 1 bis 4, wobei die Carbonsäure Benzoesäure ist.

6. Zusammensetzung nach irgendeinem der Ansprüche 1 bis 5, wobei die Zusammensetzung ferner 10 bis 80 Gew.-% anionisches Tensid umfasst.

7. Zusammensetzung nach Anspruch 6, wobei das anionische Tensid unter Alkylsulfat, Alkylsulfosuccinat, Alkylsulfoacetat, Alkylethersulfat, Isethionat, Taurat und Mischungen davon ausgewählt ist.

8. Zusammensetzung nach irgendeinem der Ansprüche 1 bis 7, wobei die Zusammensetzung ferner ein oder mehrere ätherische Öle umfasst, ausgewählt unter Thymol, Terpineol, Carvacrol, Eugenol, Menthol und Mischungen davon.

9. Zusammensetzung nach Anspruch 8, wobei die Zusammensetzung ferner ein Strukturmittel, ausgewählt unter Siliciumdioxid, Talk, Kaolinit, Bentonit, Stärke, Cellulose, Feldspat, Glimmer und Mischungen davon, umfasst.

10. Zusammensetzung nach irgendeinem der Ansprüche 1 bis 9, wobei die Zusammensetzung die Form eines Stabes hat.

11. Zusammensetzung nach irgendeinem der Ansprüche 1 bis 9, wobei die Zusammensetzung in Form eines Pulvers oder eines konzentrierten Pulvers vorliegt.

12. Nicht therapeutisches Verfahren zur Erzielung einer antimikrobiellen Wirkung, umfassend die folgenden Schritte:
a. Aufbringen der Zusammensetzung nach irgendeinem der Ansprüche 1 bis 11 auf eine Oberfläche; und
b. optional Spülen der Oberfläche, auf die die Zusammensetzung aufgebracht wurde.

13. Verfahren, wie im Anspruch 12 beansprucht, wobei der Schritt des Spülens der Oberfläche innerhalb von 5 Minuten nach dem Auftragen der Zusammensetzung auf die Oberfläche durchgeführt wird.

14. Nicht therapeutische Verwendung der Zusammensetzung, wie in irgendeinem der Ansprüche 1 bis 11 beansprucht, zur Erzielung einer antimikrobiellen Wirkung.

15. Verwendung eines Ammoniumsalzes als Verstärker der antimikrobiellen Wirksamkeit einer festen Reinigungszusammensetzung, wobei die Reinigungszusammensetzung Carbonsäure mit einem pKa-Wert im Bereich von 2,5 bis 4,5 umfasst und wobei der pH-Wert einer 5%-igen Lösung der Zusammensetzung im Bereich von 2,5 bis 6,5 liegt.

## Revendications

1. Composition nettoyante solide comprenant :
a. 2 à 15 % en poids de sel d'ammonium choisi parmi le chlorure d'ammonium, le benzoate d'ammonium, le citrate d'ammonium, le carbonate d'ammonium, l'acétate d'ammonium, le sulfate d'ammonium, le chlorure d'isopropylammonium et leurs mélanges ; et
b. 2 à 25 % en poids d'un ou plusieurs acides carboxyliques ayant un pKa situé dans la plage allant de 2,5 à 4,5 ;
dans laquelle le pH d'une solution à 5 % de la composition est situé dans la plage allant de 2,5 à 6,5.

2. Composition selon la revendication 1, dans laquelle le sel d'ammonium est choisi parmi le chlorure d'ammonium, le citrate d'ammonium et leurs mélanges.

3. Composition selon l'une quelconque des revendications 1 et 2, dans laquelle l'acide carboxylique est choisi parmi les acides carboxyliques aromatiques, les acides carboxyliques cycliques, les acides carboxyliques aliphatiques et leurs mélanges.

4. Composition selon l'une quelconque des revendications 1 à 3, dans laquelle l'acide carboxylique est choisi parmi les acides carboxyliques aromatiques choisis parmi l'acide benzoïque, l'acide 2-hydroxybenzoïque, l'acide 3-hydroxybenzoïque, et les acides carboxyliques aliphatiques choisis parmi l'acide citrique, l'acide lactique ; et leurs mélanges.

5. Composition selon l'une quelconque des revendications 1 à 4, dans laquelle l'acide carboxylique est l'acide benzoïque.

6. Composition selon l'une quelconque des revendications 1 à 5, laquelle composition comprend en outre 10 à 80 % en poids de tensioactif anionique.

7. Composition selon la revendication 6, dans laquelle le tensioactif anionique est choisi parmi un alkylsulfate, un alkylsulfosuccinate, un alkylsulfoacétate, un alkyléthersulfate, un iséthionate, un taurate, et leurs mélanges.

8. Composition selon l'une quelconque des revendications 1 à 7, laquelle composition comprend en outre une ou plusieurs huiles essentielles choisies parmi le thymol, le terpinéol, le carvacrol, l'eugénol, le menthol et leurs mélanges.

9. Composition selon la revendication 8, laquelle composition comprend en outre un structurant choisi parmi la silice, le talc, la kaolinite, la bentonite, l'amidon, la cellulose, le feldspath, le mica et leurs mélanges.

10. Composition selon l'une quelconque des revendications 1 à 9, laquelle composition est sous la forme d'un pain.

11. Composition selon l'une quelconque des revendications 1 à 9, laquelle composition est sous la forme d'une poudre ou d'une poudre concentrée.

12. Procédé non thérapeutique de fourniture d'un effet antimicrobien, comprenant les étapes suivantes :
a. application de la composition de l'une quelconque des revendications 1 à 11 sur une surface ; et
b. éventuellement rinçage de la surface sur laquelle la composition est appliquée.

13. Procédé selon la revendication 12, dans lequel l'étape de rinçage de la surface est effectuée dans les 5 minutes suivant l'application de la composition sur la surface.

14. Utilisation non thérapeutique de la composition de l'une quelconque des revendications 1 à 11 pour fournir un effet antimicrobien.

15. Utilisation d'un sel d'ammonium en tant qu'amplificateur de l'efficacité antimicrobienne d'une composition nettoyante solide, ladite composition nettoyante comprenant de l'acide carboxylique ayant un pKa situé dans la plage allant de 2,5 à 4,5 ; et dans laquelle le pH d'une solution à 5 % de la composition est situé dans la plage allant de 2,5 à 6,5.
